# EUROPEAN PATENT SPECIFICATION

(11) **EP 2 208 470 B1**
(45) Date of publication and mention of the grant of the patent: **29.02.2012**
(21) Application number: 10150487.6
(22) Date of filing: 12.01.2010
(51) Int. Cl.: A61B 17/15, A61B 19/00

(54) **Patella resectioning guide**
Patellaresektionsführung
Guide de réselection de rotule

(30) Priority: 16.01.2009 US 355235
(43) Date of publication of application: 21.07.2010
(60) Divisional application: 11184387.6
(73) Proprietor: DePuy Products, Inc., Warsaw, IN 46581 (US)
(72) Inventor: Lester, Mark B., Warsaw, IN 46580 (US); Rhodes, James M., Warsaw, IN 46582 (US)
(74) Representative: Belcher, Simon James

(56) References cited:
- WO-A1-2008/112996
- US-A- 5 108 401
- US-A1- 2005 234 461

## Description

This invention relates to orthopaedic surgical instruments, especially to a patella resectioning guide.

Joint arthroplasty is a well-known surgical procedure by which a diseased and/or damaged natural joint is replaced by a prosthetic joint. A typical knee prosthesis includes a tibial tray, a femoral component, and a polymer insert or bearing positioned between the tibial tray and the femoral component. In some cases, the knee prosthesis may also include a prosthetic patella component, which is secured to a posterior side of the patient's surgically-prepared patella. To do so, an orthopaedic surgeon may resect the posterior side of the patient's natural patella to secure the prosthetic component thereto. In use, the patella component articulates with the patient's natural or prosthetic femur during extension and flexion of the patient's knee.

To facilitate the replacement of the natural joint with the knee prosthesis, orthopaedic surgeons use a variety of orthopaedic surgical instruments such as, for example, cutting blocks, drill guides, milling guides, and other surgical instruments. Typically, the orthopaedic surgical instruments are generic with respect to the patient such that the same orthopaedic surgical instrument may be used on a number of different patients during similar orthopaedic surgical procedures.

US-A-2005/0234461 discloses instruments for use in resecting a patella which include a cutting block and a clamp. The cutting block can have a groove in its opposite face which are designed to mate with a patellar clamp. The groove is shaped to receive a ring-shaped grasper on the clamp.

The present invention provides a patella resectioning guide assembly as defined in claim 1.

In some embodiments, the cutting guide may be formed from a material different from the material forming the body. The cutting guide may be formed from a metallic material or a non-metallic material. In some embodiments, the body may be formed from a polymeric material. In such embodiments, the cutting guide may be over-moulded with the body.

The resectioning guide may further include a medial side corresponding to the medial side of the patient's patella when patella is received in the negative contour of the body. Additionally, the resectioning guide may include a lateral side corresponding to the lateral side of the patient's patella when the patella is received in the negative contour of the body. The cutting slot of the cutting guide may include a first opening on the medial side of the resectioning guide. The first opening may be sized to receive a cutting saw blade. In some embodiments, the cutting slot of the cutting guide may include a second opening on the lateral side of the resectioning guide. The second opening may also be sized to receive a cutting saw blade. Additionally, in some embodiments, the resectioning guide may include an indentation formed on the medial side of the resectioning guide. The indentation may extend from the outer surface of the body to the cutting slot of the cutting guide.

In some embodiments, the body of the resectioning guide may include a sidewall extending upwardly from the bone-facing surface and intersecting with the resectioning plane to prevent the cutting saw blade from extending beyond the body. The sidewall may be positioned on the lateral side of the resectioning guide in some embodiments. Additionally, the cutting guide may be positioned on the medial side of the resectioning guide such that the negative contour of the body may be positioned between the cutting guide and the sidewall.

Additionally the indentation may be sized to receive a thumb of a surgeon such that the body and patella may be held between the thumb and forefinger of the orthopaedic surgeon. Further, in some embodiments, the resectioning guide may further include compressible foam material secured to the outer surface of the body.

Additionally, in some embodiments, the resectioning guide may further include an enclosed housing extending upwardly from the bone-facing surface of the body. The enclosed housing may be spaced apart from the cutting guide. The enclosed housing may have an aperture co-planar with the resectioning plane defined by the cutting slot. Additionally, the enclosed housing may be positioned on the medial side of the resectioning guide, and the cutting guide may be positioned on the lateral side of the resectioning guide such that the negative contour is formed between the cutting guide and the enclosed housing.

Preferably, the resectioning guide includes an enclosed housing spaced apart from the cutting guide and extending upwardly from the bone-facing surface of the body, the enclosed housing having an aperture co-planar with the resectioning plane defined by the cutting slot. Preferably, the enclosed housing is positioned on the medial side of the resectioning guide, and the cutting guide is positioned on the lateral side of the resectioning guide such that the negative contour is formed between the cutting guide and the enclosed housing.

Embodiments of the invention are described below by way of example with reference to the accompanying drawings, in which:
FIG. 1 is a simplified flow diagram of a method for designing and fabricating a customised patient-specific patella resectioning guide;
FIG. 2 is a perspective view of one embodiment of a customised patient-specific patella resectioning guide;
FIG. 3 is another perspective view of the customised patient-specific patella resectioning guide of FIG. 2;
FIG. 4 is a perspective view the customised patient-specific patella resectioning guide of FIG. 2 with a patient's patella positioned in the customised patient-specific patella resectioning guide;
FIG. 5 is a perspective view of another embodiment of a customised patient-specific patella resectioning guide;
FIG. 6 is a side elevation view of the customised patient-specific patella resectioning guide of FIG. 5; and
FIG. 7 is a perspective view of another embodiment of a customised patient-specific patella resectioning guide.

Terms representing anatomical references, such as anterior, posterior, medial, lateral, superior, inferior, etcetera, may be used throughout the specification in reference to the orthopaedic implants and surgical instruments described herein as well as in reference to the patient's natural anatomy. Such terms have well-understood meanings in both the study of anatomy and the field of orthopaedics. Use of such anatomical reference terms in the written description and claims is intended to be consistent with their well-understood meanings unless noted otherwise.

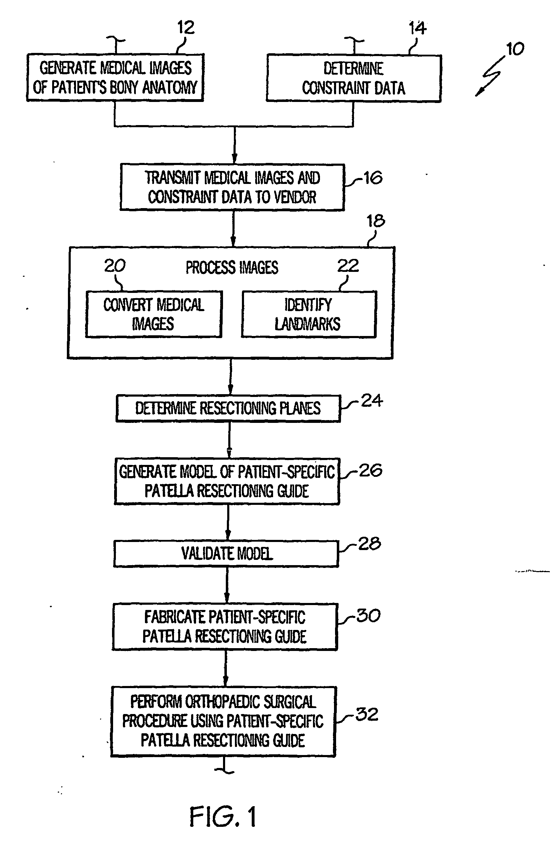

Referring to the drawings, FIG. 1 is a flow diagram with details of a method 10 which can be used to fabricate a customised patient-specific orthopaedic surgical instrument. The term "customised patient-specific orthopaedic surgical instrument" is used in this document to refer to a surgical tool for use by a surgeon in performing an orthopaedic surgical procedure that is intended, and configured, for use on a particular patient. A "customised patient-specific orthopaedic surgical instrument" is distinct from a standard, non-patient specific orthopaedic surgical instrument which is intended for use on a variety of different patients. Additionally, it should be appreciated that the term "customised patient-specific orthopaedic surgical instrument" is distinct from orthopaedic prostheses, whether patient-specific or generic, which are surgically implanted in the body of the patient. Rather, customised patient-specific orthopaedic surgical instruments are used by an orthopaedic surgeon to assist in the implantation of orthopaedic prostheses.

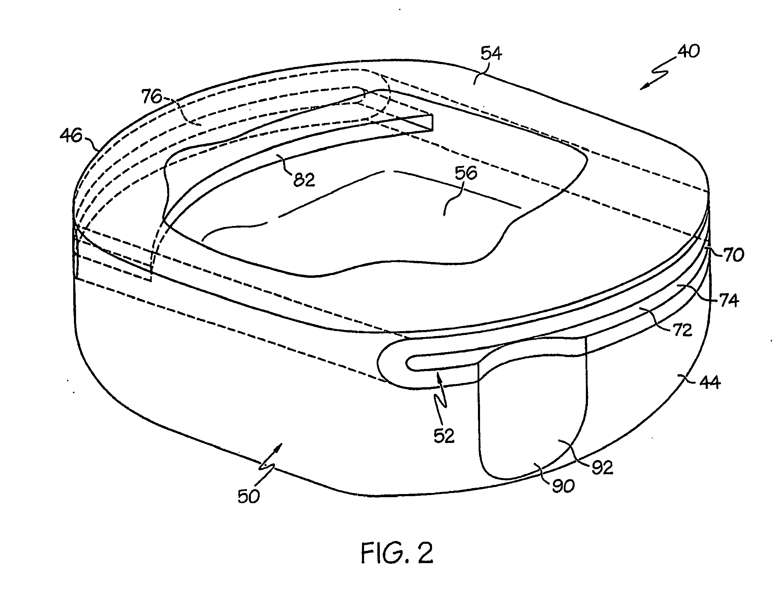

In some embodiments, the customised patient-specific orthopaedic surgical instrument may be customised to the particular patient based on the location at which the instrument is to be coupled to one or more bones of the patient. For example, in some embodiments, the customised patient-specific orthopaedic instrument may be a customised patient-specifc patella resectioning guide including one or more bone-contacting or facing surfaces having a negative contour that matches the contour of a portion of the patient's patella, which is discussed in more detail below with reference to FIGS. 2 to 7. As such, the customised patient-specific patella resectioning guide is configured to be coupled to the patient's patella at a unique location and position with respect to the patient's bony anatomy. That is, the negative contours of the bone-contacting surfaces are configured to receive a matching contour surface of the portion of the patient's patella (and, in some cases, femur). As such, the orthopaedic surgeon's guesswork and/or intra-operative decision-making with respect to the placement of the patient-specific patella resectioning guide are reduced. The orthopaedic surgeon may simply couple the customised patient-specific patella resectioning guide to the patient's patella. When so coupled, the customised patient-specific patella resectioning guide defines the thickness of bone the surgeon will resect from the posterior side of the patient's patella.

As shown in FIG. 1, the method 10 includes process steps 12, 14 in which an orthopaedic surgeon performs pre-operative planning of the patella resectioning procedure to be performed on a patient. The process steps 12, 14 may be performed in any order or contemporaneously with each other. In process step 12, a number of medical images of the patient's patella and the surrounding bony anatomy are generated. To do so, the orthopaedic surgeon or other healthcare provider may operate an imaging system to generate the medical images. The medical images may be embodied as any number and type of medical images capable of being used to generate a three-dimensional rendered model of the patient's patella and surrounding bony anatomy. For example, the medical images may be embodied as any number of computed tomography (CT) images, magnetic resonance imaging (MRI) images, or other three-dimensional medical images. Additionally, or alternatively, as discussed in more detail below in regard to process step 18, the medical images may be embodied as a number of X-ray images or other two-dimensional images from which a three-dimensional rendered model of the patient's patella and the surrounding bony anatomy may be generated.

In process step 14, the orthopaedic surgeon may determine any additional pre-operative constraint data. The constraint data may be based on the orthopaedic surgeon's preferences, preferences of the patient, anatomical aspects of the patient, guidelines established by the healthcare facility, or the like. For example, the constraint data may include the orthopaedic surgeon's preference for a particular prosthesis type, the thickness of the bone to resect, the size range of the orthopaedic implant, and/or the like. In some embodiments, the orthopaedic surgeon's preferences are saved as a surgeon's profile, which may be used as a default constraint values for further surgical plans.

In process step 16, the medical images and the constraint data, if any, are transmitted or otherwise provided to an orthopaedic surgical instrument vendor or manufacturer. The medical images and the constraint data may be transmitted to the vendor via electronic means such as a network or the like. After the vendor has received the medical images and the constraint data, the vendor processes the images in step 18. The orthopaedic surgical instrument vendor or manufacturer process the medical images to facilitate the determination of the proper positioning of the prosthetic component, implant sizing, and fabrication of the customised patient-specific patella resectioning guide as discussed in more detail below.

In process step 20, the vendor may convert or otherwise generate three-dimensional images from the medical images. For example, in embodiments wherein the medical images are embodied as a number of two-dimensional images, the vendor may use a suitable computer algorithm to generate one or more three-dimensional images from the number of two-dimensional images. Additionally, in some embodiments, the medical images may be generated based on an established standard such as the Digital Imaging and Communications in Medicine (DICOM) standard. In such embodiments, an edge-detection, thresholding, watershed, or shape-matching algorithm may be used to convert or reconstruct images to a format acceptable in a computer aided design application or other image processing application. Further, in some embodiments, an algorithm may be used to account for tissue such as cartilage not discernable in the generated medical images. In such embodiments, any three-dimensional model of the patient-specific instrument (see, e.g., process step 26 below) may be modified according to such algorithm to increase the fit and function of the instrument.

In process step 22, the vendor may process the medical images, and/or the converted/reconstructed images from process step 20, to determine a number of aspects related to the bony anatomy of the patient such as the anatomical axis of the patient's bones, the mechanical axis of the patient's bone, other axes and various landmarks, and/or other aspects of the patient's bony anatomy. To do so, the vendor may use any suitable algorithm to process the images.

The resectioning plane of the patient's patella is determined in process step 24. The planned resectioning plane is determined based on the type, size, and position of the prosthetic patella component to be used during the orthopaedic procedure, on the process images such as specific landmarks identified in the images, and on the constraint data supplied by the orthopaedic surgeon in previous process steps 14, 16. The type and/or size of the prosthetic patella component may be determined based on the patient's anatomy and the constraint data. For example, the constraint data may dictate the type, make, model, size, or other characteristic of the prosthetic patella component. The selection of the prosthetic patella component may also be modified based on the medical images such that a prosthetic component usable with the bony anatomy of the patient and matching the constraint data or preferences of the orthopaedic surgeon is selected.

In addition to the type and size of the prosthetic patella component, the planned location and position of the prosthetic patella component relative to the patient's bony anatomy is determined. To do so, a digital template of the prosthetic patella component may be overlaid onto one or more of the processed medical images. The vendor may use any suitable algorithm to determine a recommended location and orientation of the prosthetic patella component (i.e., the digital template) with respect to the patient's bone based on the processed medical images (e.g., landmarks of the patient's patella and/or femur defined in the images) and/or the constraint data. Additionally, any one or more other aspects of the patient's bony anatomy may be used to determine the proper positioning of the digital template. In some embodiments, the digital template along with surgical alignment parameters may be presented to the orthopaedic surgeon for approval.

The planned resectioning planes for the patient's patella may then be determined based on the determined size, location, and orientation of the prosthetic patella component. In addition, other aspects of the patient's bony anatomy, as determined in process step 22, may be used to determine or adjust the planned resectioning planes. For example, the determined mechanical axis, landmarks, and/or other determined aspects of the femur and/or patella of the patient may be used to determine the planned resectioning planes.

In process step 26, a model of the customised patient-specific patella resectioning guide is generated. In some embodiments, the model is embodied as a three-dimensional rendering of the customised patient-specific patella resectioning guide. In other embodiments, the model may be embodied as a mock-up or fast prototype of the customised patient-specific patella resectioning guide. The customised patient-specific patella resectioning guide to be modelled and fabricated may be determined based on the patella orthopaedic surgical procedure to be performed, the constraint data, and/or the type of prosthetic patella component to be implanted in the patient.

The particular shape of the customised patient-specific patella resectioning guide is determined based on the planned location of the patella resectioning guide relative to the patient's bony anatomy. The location of the customised patient-specific patella resectioning guide with respect to the patient's bony anatomy is determined based on the type and determined location of the prosthetic patella component to be used during the orthopaedic surgical procedure. That is, the planned location of the customised patient-specific patella resectioning guide relative to the patient's bony anatomy may be selected based on, in part, the planned resectioning planes of the patient's bone(s) as determined in step 24. For example, the location of the patella resectioning guide is selected such that the cutting guide of the patella resectioning guide matches one or more of the planned resectioning planes determined in process step 24. Additionally, the planned location of the patella resectioning guide may be based on the identified landmarks of the patient's patella and femur identified in process step 22.

In some embodiments, the particular shape or configuration of the customised patient-specific patella resectioning guide may be determined based on the planned location of the guide relative to the patient's bony anatomy. That is, the customised patient-specific patella resectioning guide may include a bone-contacting surface having a negative contour that matches a corresponding positive contour of a portion of the patella of the patient. The positive contour of the portion of the patella of the patient may be received in the negative contour of the patella resectioning guide such that the patella is placed in a unique location. When the patella resectioning guide receives the patient's patella, one or more guides (e.g., cutting guide) of the patella resectioning guide may be aligned to the one or more of the resectioning plane(s), as discussed above.

After the model of the customised patient-specific patella resectioning guide has been generated in process step 26, the model is validated in process step 28. The model may be validated by, for example, analysing the rendered model while the three-dimensional model of the patient's patella is received in the resectioning guide model to verify the correlation of the cutting guide and the resectioning plane. Additionally, the model may be validated by transmitting or otherwise providing the model generated in step 26 to the orthopaedic surgeon for review. For example, in embodiments wherein the model is a three-dimensional rendered model, the model along with the three-dimensional images of the patient's relevant bone(s) may be transmitted to the surgeon for review. In embodiments wherein the model is a physical prototype, the model may be shipped to the orthopaedic surgeon for validation.

After the model has been validated in process step 28, the customised patient-specific patella resectioning guide is fabricated in process step 30. The customised patient-specific patella resectioning guide may be fabricated using any suitable fabrication device and method. Additionally, the customised patient-specific patella resectioning guide may be formed from any suitable material such as a metallic material, a plastic material, or combination thereof depending on, as discussed in more detail below. The fabricated customised patient-specific patella resectioning guide is subsequently shipped or otherwise provided to the orthopaedic surgeon. The surgeon performs the orthopaedic surgical procedure in process step 32 using the customised patient-specific patella resectioning guide. As discussed above, because the orthopaedic surgeon does not need to determine the proper location of the patella resectioning guide intra-operatively, which typically requires some amount of estimation on part of the surgeon, the guesswork and/or intra-operative decision-making on part of the orthopaedic surgeon is reduced.

It should also be appreciated that variations in the bony anatomy of the patient may require more than one customised patient-specific patella resectioning guide to be fabricated according to the method described herein. For example, the patient may require the implantation of two prosthetic patella components to replace both natural knees. As such, the surgeon may follow the method 10 of FIG. 1 to fabricate a different customised patient-specific patella resectioning guide for use in replacing each natural knee. Each customised patient-specific patella resectioning guide defines a particular resectioning plane relative to each particular patella that is different due to the variation in the bony anatomy of each knee joint.

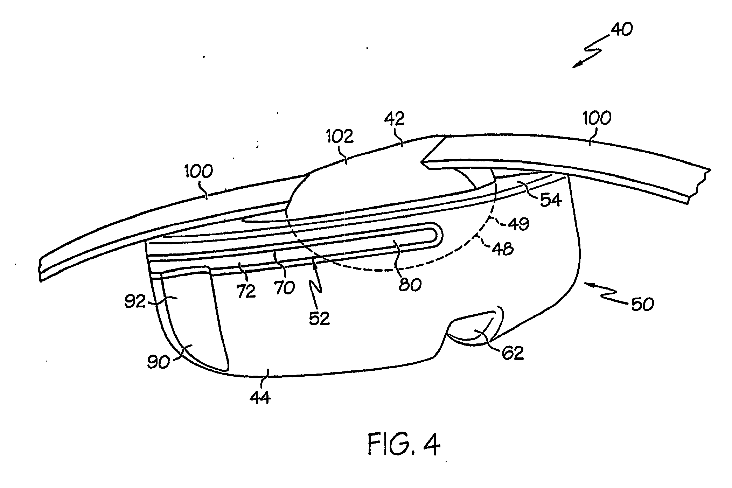

FIGS. 2 to 4 show an embodiment of a customised patient-specific patella resectioning guide 40 which is configured to receive a portion of a posterior side 48 of a patient's patella 42 (see FIG. 4). The resectioning guide 40 has a medial side 44 and a lateral side 46. The medial side 44 corresponds to the medial side of the patient's patella 42 when the patella 42 is received in the resectioning guide 40. Similarly, the lateral side 46 corresponds to the lateral side of the patient's patella 42 when the patella 42 is received in the resectioning guide 40. The orthopaedic surgeon may use the patella resectioning guide 40 to make a resectioning cut of the patient's patella 42. A prosthetic patella component may be subsequently secured to the resected surface of the posterior side 48 of the patient's patella 42.

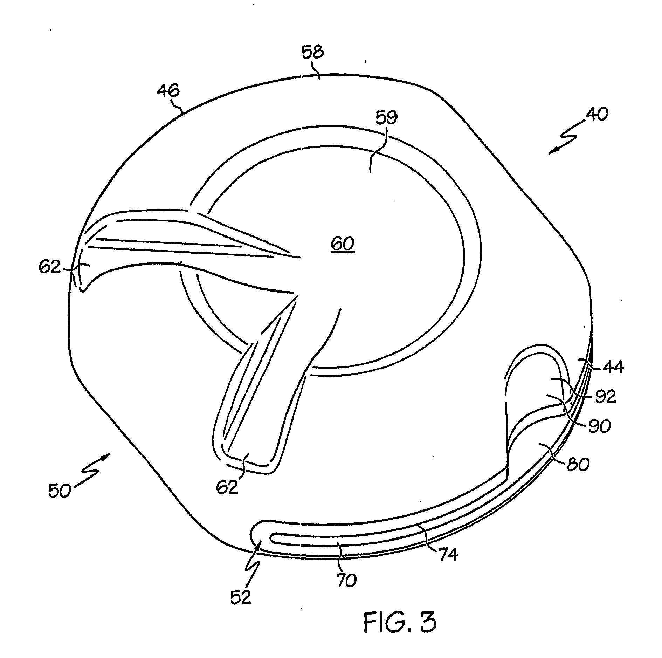

As shown in FIGS. 2 and 3, the patella resectioning guide 40 includes a body 50 and a captured cutting guide 52, which is secured to the body 50. The body 50 is formed from a polymeric material such as polyethylene or ultra-high molecular weight polypropylene (UHMWPP), and the cutting guide 52 is formed from an implant grade metallic material such as steel, titanium, or cobalt chromium. However, in other embodiments, the cutting guide 52 may also be formed from a polymeric material. That is, the body 50 and the cutting guide 52 may be formed from a single monolithic component. As such, the body 50 and the cutting guide 52 may be made of the same or different materials.

In the embodiment of FIGS. 2 to 4, the body 50 includes a bone-facing surface 54 and an outer surface 58 opposite the bone-facing surface 54. The bone-facing surface 54 extends from the medial side 44 of the resectioning guide 40 to the lateral side 46. The bone-facing surface 54 includes a customised patient-specific negative contour 56 defined therein. The negative contour 56 is configured to receive a corresponding positive contour 49 of the posterior side 48 of the patient's patella 42. As discussed above, the negative contour 56 of the bone-facing surface 54 allows the surgeon to position the patient's patella 42 in the resectioning guide 40 in a unique, pre-determined location and orientation.

As shown in FIG. 3, the outer surface 58 includes a recess 59 shaped to receive an end of a clamp (not shown) or other tool for holding the patient's patella in the resectioning guide 40. As shown in FIG. 3, the recess 59 has a round indentation 60 and a pair of slots 62 formed in the outer surface 58. The round indentation 60 and the slots 62 are sized to receive the clamp holding the patella 42 to the body 50. It should be appreciated that in other embodiments the recess 59 may include an indentation 60 formed as square, triangular, or any other form suitable to receive the clamp. Similarly, it should be appreciated that the recess 59 may include additional slots of differing sizes as necessary to receive the clamp. Additionally, in some embodiments, the surgeon may secure the patella 42 to the body 50 by fixing the patella 42 and body 50 between two fingers. In such embodiments, the recess 59 is sized to receive one of the surgeon's fingers therein.

The cutting guide 52 includes a cutting slot 70 extending from the medial side 44 of the resectioning guide 40 to the lateral side 46 of the resectioning guide 40. The cutting slot 70 defines a resectioning plane 72 that extends through the patient's patella 42 when the patella 42 is received in the customised patient-specific contour 56 of the body 50. The cutting slot 70 extends from a medial opening 74 formed in the medial side 44 through an opening (not shown) into the negative contour 56 of the body 50. The cutting slot 70 also extends from a lateral opening 76 formed in the lateral side 46 through an opening 82 into the negative contour 56 of the body 50. The cutting slot 70 is sized to receive a cutting saw blade (not shown).

A viewing window 90 is formed on the medial side 44 of the resectioning guide 40. The viewing window 90 is formed as an indentation 92 extending from the outer surface 58 of the body 50 to the interior of the cutting slot 70 of the cutting guide 52. The viewing window 90 is positioned to allow the surgeon to view the cutting saw blade within the cutting slot 70. It should be appreciated that in other embodiments the body 50 and cutting guide 52 may include additional viewing windows of different sizes and shapes to facilitate the surgeon's view of the cutting saw blade. For example, in one embodiment, the resectioning guide 40 may include a viewing window on each of the medial and lateral sides 44, 46.

As shown in FIG. 4, in one embodiment, the patella 42 remains secured to the tendons 100 of the patient during the resectioning procedure. The tendons 100 may contact the bone-facing surface 54 of the body 50 when the positive contour 49 of the posterior side 48 of the patient's patella 42 is received in the negative contour 56. As such, in some embodiments, the body 50 may include indentations in the bone facing surface 54 to provide space to receive a portion of the tendons 100.

In use, an orthopaedic surgeon may secure the patella 42 to the body 50 using the clamp or other securing means. The surgeon may then insert the cutting saw blade into the opening 76. The blade passes through the cutting slot 70 and contacts the medial side of the portion of the patella 42 received in the negative contour 56. Following the resectioning plane 72 defined by the cutting slot 70, the surgeon may perform the resectioning cut on the patient's patella 42 by moving the saw blade back and forth within cutting slot 70. As the blade cuts through the patella 42, the blade is received in the opening 82 of the cutting slot 70. In this way, the blade is captured on both the medial side and the lateral side of the patient's patella 42.

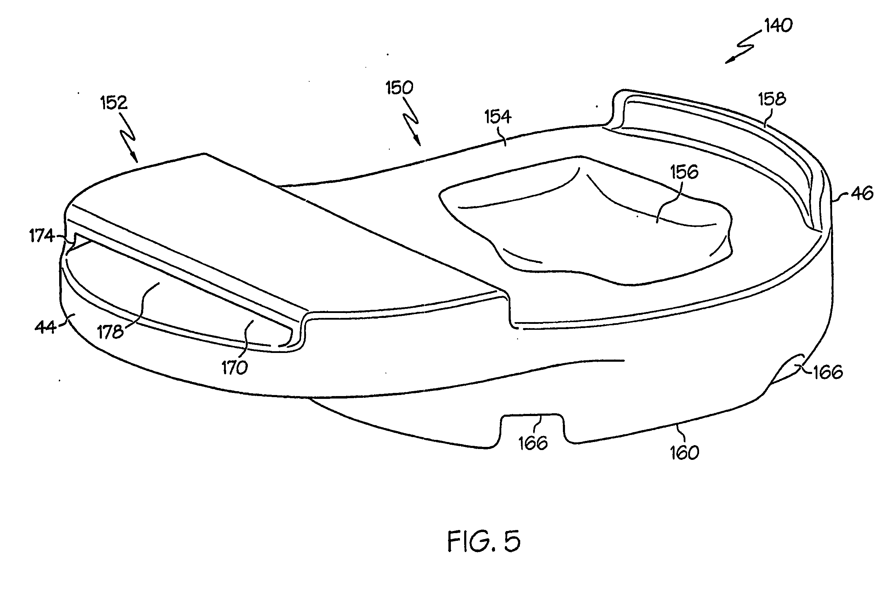

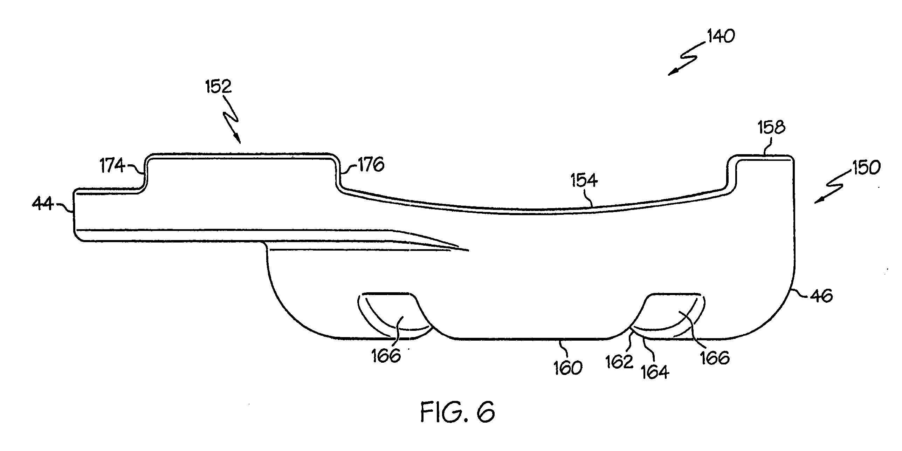

FIGS. 5 and 6 show another embodiment of patella resectioning guide 140. Similar to the embodiment of FIGS. 2 to 4, the resectioning guide 140 has a medial side 44 and a lateral side 46. The resectioning guide 140 also includes a body 150 and a cutting guide 152, which is secured to the body 150 and positioned on the medial side 44 of the resectioning guide 140. The resectioning guide 140 is formed as a single monolithic component from a polymeric material such as those discussed above with reference to the embodiment of FIGS. 2 to 4.

The body 150 includes a bone-facing surface 154 having a customised patient-specific negative contour 156 defined therein. Similar to the negative contour 56, the negative contour 156 is configured to receive the corresponding positive contour 49 of the posterior side 48 of the patient's patella 42. The body 150 also includes a sidewall 158 extending upwardly from the bone-facing surface 154. The sidewall 158 is positioned on the lateral side 46 of the resectioning guide 140 such that the customised patient-specific contour 156 is positioned between the sidewall 158 and the cutting guide 152.

Similar to the embodiment of FIGS. 2 to 4, the body 150 also includes an outer surface 160 opposite the bone-facing surface 154. The outer surface 160 includes a recess 162 shaped to receive an end of a clamp (not shown) or other tool for holding the patient's patella 42 in the resectioning guide 140. The recess 162 has a round indentation 164 and a pair of slots 166 formed in the outer surface 160. The round indentation 164 and the slots 166 are sized to receive the clamp (not shown) operable to secure the patella 42 to the body 150. It should be appreciated that the recess 162 includes indentations and slots for use in holding the patient's patella 42 in the resectioning guide 140.

The cutting guide 152 includes a cutting slot 170 sized to receive a cutting saw blade (not shown). The cutting slot 170 extends from an opening 174 defined in the medial side 44 of the resectioning guide 140 to an opening (not shown) in a bone-facing surface 176 of the cutting guide 152. The cutting slot 170 defines a resectioning plane 178 extending through the patient's patella 42 when the patella 42 is received in the customised patient-specific negative contour 156 of the body 150. The sidewall 158 intersects with the resectioning plane 178 and extends upwardly from the body 150 a length sufficient to prevent a cutting saw blade (not shown) from extending beyond the lateral side 46 of the resectioning guide 140 during use.

Similar to the embodiment of FIGS. 2 to 4, the tendons 100 of the patient may contact the bone-facing surface 154 of the body 150 when the positive contour 49 of the posterior side 48 of the patient's patella 42 is received in the negative contour 156. To provide additional space for the tendons 100, the bone-facing surface 154 has a medial-to-lateral concave contour (see FIG. 6) which is configured to receive a portion of tendons 100.

In use, an orthopaedic surgeon may secure the patella 42 to the body 150 using the clamp or other securing means. The surgeon may then insert the cutting saw blade into the opening 174. The blade passes through the cutting slot 170 and contacts the medial side of the portion of the patella 42 received in the negative contour 156. Following the resectioning plane 178 defined by the cutting slot 170, the surgeon may perform a resectioning cut on the patient's patella 42 by moving the blade back and forth within the cutting slot 170. In this way, the blade is captured within the cutting guide 152 while the surgeon performs the resectioning cut. As the blade cuts through the patella 42, the sidewall 158 prevents the surgeon from pushing the blade beyond the resectioning guide 140.

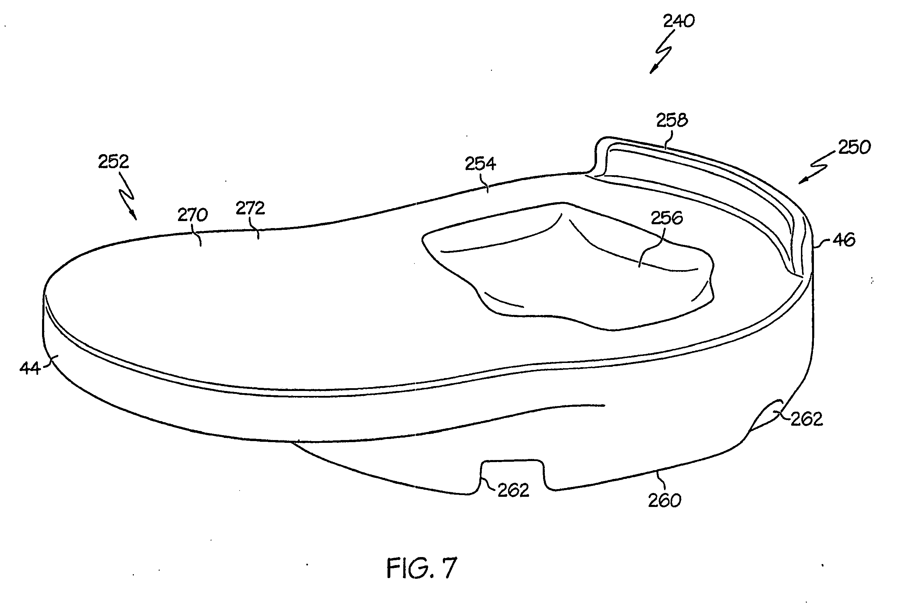

Referring now to FIG. 7, in another embodiment, the customised patient-specific patella resectioning guide may be embodied as a patella resectioning guide 240. Similar to the embodiments of FIGS. 2 to 6, the resectioning guide 240 has a medial side 44 and a lateral side 46. The resectioning guide 240 also includes a body 250 and a non-captured cutting guide 252 that is secured to the body 250 and formed on the medial side 44. Like the embodiment of FIGS. 5 and 6, the patella resectioning guide 240 is formed as a single monolithic component.

The body 250 includes a bone-facing surface 254 having a customised patient-specific negative contour 256 defined therein. The negative contour 256 is configured to receive the corresponding positive contour 49 of the posterior side 48 of the patient's patella 42. Similar to the resectioning guide 140 of FIGS. 5 and 6, the body 250 also includes a sidewall 258 extending upwardly from the bone-facing surface 254. The sidewall 258 is positioned on the lateral side 46 of the resectioning guide 240 such that the customised patient-specific negative contour 256 is positioned between the cutting guide 252 and the sidewall 258.

Similar to the embodiment of FIGS. 2 to 6, the body 250 also includes an outer surface 260 opposite the bone-facing surface 254. The outer surface 260 includes a recess 262 shaped to receive an end of a clamp (not shown) or other tool for holding the patient's patella in the resectioning guide 240. It should be appreciated that the recess 262 includes indentations and slots for use in holding the patient's patella 42 in the resectioning guide 240.

The non-captured cutting guide 252 includes a cutting surface 270 and the bone-facing surface 254. The cutting surface 270 extends from the medial side 44 to the bone-facing surface 254 of the body 250. That is, the cutting surface 270 and the bone-facing surface 254 are co-planar with each other. The cutting surface 270 defines a resectioning plane 272 that the surgeon follows to perform a resectioning cut on the patient's patella 42 when the patella 42 is received in the customised patient-specific negative contour 256 of the body 250. The sidewall 258 intersects with the resectioning plane 272 and extends upwardly from the body 150 a length sufficient to prevent a cutting saw blade (not shown) from extending beyond the lateral side 46 of the resectioning guide 240 during use.

## Claims

1. A patella resectioning guide assembly which comprises a patella resectioning guide (40; 140; 240) and a clamp, in which the patella resectioning guide comprises:
(i) a body (50; 150; 250) including a bone-facing surface (54) having a negative contour (56) configured to receive a portion of a posterior side of a patient's patella (42) which has a corresponding positive contour, and an outer surface (58; 160; 260) opposite to the bone-facing surface which has an indentation (60 ; 162 ; 262) formed in it, and
(ii) a cutting guide (52 ; 152 ; 252) coupled to the body which includes a cutting slot (70 ; 170) defined therein or a non-captured cutting surface (270), the cutting slot or the cutting surface defining a resectioning plane (72 ; 272), the cutting guide being positioned such that the resectioning plane extends through the patient's patella when the patella is received in the negative contour of the body,
in which the clamp has an end which can be received in the indentation when used to hold the patient's patella in the negative contour of the body,
**characterised in that** the outer surface of the body has a pair of grooves (62 ; 166 ; 262) extending between the indentation and the edge of the outer surface which are sized to receive the clamp when the end of the clamp is received in the indentation.

2. An assembly as claimed in claim 1, in which the body (50) is formed of a polymeric material, and the cutting guide (52) is over-moulded with the body of the patella resectioning guide.

3. An assembly as claimed in claim 1, which has:
a medial side (44) corresponding to the medial side of the patient's patella (42) when the patella is received in the negative contour (56) of the body (50), and
a lateral side (46) corresponding to the lateral side of the patient's patella when the patella is received in the negative contour of the body,
in which the cutting slot (70) of the cutting guide includes a first opening (74) on the medial side of the resectioning guide (40), the first opening being sized to receive a cutting saw blade.

4. An assembly as claimed in claim 3, in which the cutting slot (70) of the cutting guide (52) includes a second opening (76) on the lateral side of the resectioning guide (40), the second opening being sized to receive a cutting saw blade.

5. An assembly as claimed in claim 3, which has an indentation (92) formed on the medial side (44) of the resectioning guide (40), and in which the indentation extends from the outer surface (58) of the body (50) to the cutting slot (70) of the cutting guide (52).

6. An assembly as claimed in claim 3, in which the body (150) includes a sidewall (158) extending upwardly from the bone-facing surface (154) and intersecting with the resectioning plane (178) to prevent the cutting saw blade from extending beyond the body.

7. An assembly as claimed in claim 6, in which:
the sidewall (158) is positioned on the lateral side (46) of the resectioning guide (140), and
the cutting guide (152) is positioned on the medial side (52) of the resectioning guide such that the negative contour (56) of the body is positioned between the cutting guide and the sidewall.

8. An assembly as claimed in claim 1, in which the body (50) and the cutting guide (52) are formed as a single monolithic component.

## Patentansprüche

1. Anordnung mit einer Patellaresektionsfuhrung, die eine Patellaresektionsführung (40; 140; 240) und eine Klammer aufweist, wobei die Patellaresektionsführung aufweist:
(i) einen Körper (50; 150; 250), der eine zum Knochen weisende Fläche (54) mit einer negativen Kontur (56), die so ausgelegt ist, dass sie einen Bereich einer hinten liegenden Seite der Patella (42) eines Patienten aufnimmt, welcher eine entsprechende positive Kontur hat, und eine der zum Knochen weisenden Fläche gegenüberstehende Außenfläche (58; 160; 260), die eine darin gebildete Vertiefung (60; 162; 262) hat, umfasst und
(ii) eine Schneidführung (52; 152; 252), die an den Körper gekoppelt ist, die einen Schneidschlitz (70; 170), der darin definiert ist, oder eine nicht greifende Schneidfläche (270) umfasst, wobei der Schneidschlitz oder die Schneidfläche eine Resektionsebene (72; 272) definieren, wobei die Schneidfläche derart angeordnet ist, dass die Resektionsebene sich durch die Patella des Patienten erstreckt, wenn die Patella in der negativen Kontur des Körpers aufgenommen ist,
wobei die Klammer ein Ende hat, das in der Vertiefung aufgenommen werden kann, wenn sie verwendet wird, um die Patella des Patienten in der negativen Kontur des Körpers zu halten,
**dadurch gekennzeichnet, dass** die Außenfläche des Körpers ein Paar sich zwischen der Vertiefung und der Kante der Außenfläche erstreckende Nuten (62; 166; 262) hat, die so bemessen sind, dass sie die Klammer aufnehmen, wenn das Ende der Klammer in der Vertiefung aufgenommen ist.

2. Anordnung nach Anspruch 1, bei der der Körper (50) aus einem polymeren Material gebildet ist und die Schneidführung (52) über den Körper der Patellaresektionsführung geformt ist.

3. Anordnung nach Anspruch 1, mit:
einer medialen Seite (44), die der medialen Seite der Patella (42) des Patienten entspricht, wenn die Patella in der negativen Kontur (56) des Körpers (50) aufgenommen ist, und
eine laterale Seite (46), die der lateralen Seite der Patella des Patienten entspricht, wenn die Patella in der negativen Kontur des Körpers aufgenommen ist,
wobei der Schneidschlitz (70) der Schneidführung eine erste Öffnung (74) auf der medialen Seite der Resektionsführung (40) umfasst, wobei die erste Öffnung so bemessen ist, dass sie ein Schneidsägeblatt aufnimmt.

4. Anordnung nach Anspruch 3, bei der der Schneidschlitz (70) der Schneidführung (52) eine zweite Öffnung (76) auf der lateralen Seite der Resektionsführung (40) umfasst, wobei die zweite Öffnung so bemessen ist, dass sie ein Schneidsägeblatt aufnimmt.

5. Anordnung nach Anspruch 3, die eine Vertiefung (92) hat, welche auf der medialen Seite (44) der Resektionsführung (40) gebildet ist, und bei der sich die Vertiefung von der Außenfläche (58) des Körpers (50) zu dem Schneidschlitz (70) der Schneidführung (52) erstreckt.

6. Anordnung nach Anspruch 3, bei der der Körper (150) eine Seitenwand (158) umfasst, die sich von der zum Knochen weisenden Fläche (154) nach oben erstreckt und die Resektionsebene (178) schneidet, um zu verhindern, dass sich das Schneidsägeblatt über den Körper hinaus erstreckt.

7. Anordnung nach Anspruch 6, bei der:
die Seitenwand (158) auf der lateralen Seite (46) der Resektionsführung (140) angeordnet ist und
die Schneidführung (152) auf der medialen Seite (52) der Resektionsführung derart angeordnet ist, dass die negative Kontur (56) des Körpers sich zwischen der Schneidführung und der Seitenwand befindet.

8. Anordnung nach Anspruch 1, bei der der Körper (50) und die Schneidführung (52) als eine einzige monolithische Komponente gebildet sind.

## Revendications

1. Ensemble de guide de résection de rotule qui comprend un guide de résection de rotule (40 ; 140 ; 240) et un clamp, dans lequel le guide de résection de rotule comprend :
(i) un corps (50 ; 150 ; 250) comprenant une surface orientée vers l'os (54) ayant un contour négatif (56) configuré pour recevoir une partie d'un côté postérieur de la rotule (42) d'un patient qui a un contour positif correspondant, et une surface externe (58 ; 160 ; 260) opposée à la surface orientée vers l'os qui a une indentation (60 ; 162 ; 262) formée dans cette dernière, et
(ii) un guide de coupe (52 ; 152 ; 252) couplé au corps qui comprend une fente de coupe (70 ; 170) définie dans ce dernier ou une surface de coupe non captive (270), la fente de coupe ou la surface de coupe définissant un plan de résection (72 ; 272), le guide de coupe étant positionné de sorte que le plan de résection s'étend à travers la rotule du patient lorsque la rotule est reçue dans le contour négatif du corps,
dans lequel le clamp a une extrémité qui peut être reçue dans l'indentation lorsqu'il est utilisé pour maintenir la rotule du patient dans le contour négatif du corps,
**caractérisé en ce que** la surface externe du corps a une paire de rainures (62 ; 166 ; 262) s'étendant entre l'indentation et le bord de la surface externe qui sont dimensionnées pour recevoir le clamp lorsque l'extrémité du clamp est reçue dans l'indentation.

2. Ensemble selon la revendication 1, dans lequel le corps (50) est formé avec un matériau polymère, et le guide de coupe (52) est surmoulé avec le corps du guide de résection de rotule.

3. Ensemble selon la revendication 1, qui a :
un côté médial (44) correspondant au côté médial de la rotule (42) du patient lorsque la rotule est reçue dans le contour négatif (56) du corps (50), et
un côté latéral (46) correspondant au côté latéral de la rotule du patient lorsque la rotule est reçue dans le contour négatif du corps,
dans lequel la fente de coupe (70) du guide de coupe comprend une première ouverture (74) sur le côté médial du guide de résection (40), la première ouverture étant dimensionnée pour recevoir une lame de scie de coupe.

4. Ensemble selon la revendication 3, dans lequel la fente de coupe (70) du guide de coupe (52) comprend une deuxième ouverture (76) sur le côté latéral du guide de résection (40), la deuxième ouverture étant dimensionnée pour recevoir une lame de scie de coupe.

5. Ensemble selon la revendication 3, qui a une indentation (92) formée sur le côté médial (44) du guide de résection (40), et dans lequel l'indentation s'étend à partir de la surface externe (58) du corps (50) jusqu'à la fente de coupe (70) du guide de coupe (52).

6. Ensemble selon la revendication 3, dans lequel le corps (150) comprend une paroi latérale (158) s'étendant vers le haut à partir de la surface orientée vers l'os (154) et coupant le plan de résection (178) pour empêcher la lame de scie de coupe de s'étendre au-delà du corps.

7. Ensemble selon la revendication 6, dans lequel :
la paroi latérale (158) est positionnée sur le côté latéral (46) du guide de résection (140), et
le guide de coupe (152) est positionné sur le côté médial (52) du guide de résection de sorte que le contour négatif (56) du corps est positionné entre le guide de coupe et la paroi latérale.

8. Ensemble selon la revendication 1, dans lequel le corps (50) et le guide de coupe (52) sont formés comme un composant monolithique unique.
